## Europäisches Patentamt
## European Patent Office
## Office européen des brevets

(19)

(11) Publication number: **0 018 117**
**B1**

(12)
# EUROPEAN PATENT SPECIFICATION

(45) Date of publication of patent specification: **30.03.83**

(51) Int. Cl.³: **B 01 J 29/28, C 07 C 1/20**

(21) Application number: **80300997.6**

(22) Date of filing: **31.03.80**

(54) Catalytic production of hydrocarbons including catalyst regeneration.

(30) Priority: **04.04.79 US 27186**

(43) Date of publication of application:
**29.10.80 Bulletin 80/22**

(45) Publication of the grant of the patent:
**30.03.83 Bulletin 83/13**

(84) Designated Contracting States:
**BE DE FR GB IT NL**

(56) References cited:
**FR - A - 2 240 281**
**US - A - 4 100 262**
**US - A - 4 101 595**

(73) Proprietor: **MOBIL OIL CORPORATION**
**150 East 42nd Street**
**New York New York 10017 (US)**

(72) Inventor: **Chen, Nai-Yuen**
**Forrest Central Drive R.D.**
**Titusville New Jersey (US)**
Inventor: **Miale, Joseph Nicolas**
**25 Merritt Drive**
**Lawrenceville New Jersey (US)**
Inventor: **Reagan, William Joseph**
**10 Manor Drive**
**Englishtown New Jersey (US)**

(74) Representative: **Cooper, John Anthony**
**Mobil Court 3 Clements Inn**
**London WC2A 2EB (GB)**

Courier Press, Leamington Spa, England.

Catalytic production of hydrocarbons including catalyst regeneration

This invention relates generally to the conversion of lower alcohols and their corresponding ethers into higher carbon-number hydrocarbons over cobalt-containing zeolite catalysts, and is concerned especially with the regeneration of such catalysts.

A remarkable growth in the production of synthetic fibers, plastics and rubber has taken place in recent decades. This growth, to a very large extent, has been supported and encouraged by an expanding supply of inexpensive petrochemical raw materials such as ethylene, benzene, toluene, and xylenes. Side by side with this remarkable development, there has been an increasing demand for aromatic hydrocarbons for use as high octane gasoline components. Environmental factors which limit the lead content of gasoline are likely to aggravate the need for aromatics.

Burgeoning demand for olefins, particularly ethylene, and for aromatic hydrocarbons, has of course led to periods of shortage, either due to short supply of suitable feedstocks or to limited processing capacity. In any case, it would appear desirable to provide efficient means for converting raw materials other than petroleum to olefins and aromatic hydrocarbons.

U.S. Patents Nos. 3,894,106, 3,894,107 and 3,907,915, respectively, disclose the conversion of alcohols, ethers, carbonyls, or mixtures thereof to higher carbon number hydrocarbons by contact with a catalyst comprising a crystalline aluminosilicate zeolite having a silica to alumina ratio of at least about 12 and a Constraint Index of about 1 to 12.

The conversion of methanol and dimethyl ether to hydrocarbon is described in U.S. Patent No. 3,911,041.

All of the foregoing patents illustrate recently developed art for efficiently converting oxygenated compounds to hydrocarbons and steam using a zeolite catalyst exemplified by ZSM-5.

More recently, there has been described in U.S. Patent 4,100,262 a catalyst comprising zeolite ZSM-5 and cobalt by virtue of the zeolite being prepared in the presence of a tetraureacobalt-II salt. This catalyst is stated to be useful in the conversion of organic compounds in general and describes particularly the conversion of methanol into a gasoline product.

Catalysts of the ZSM-5 type are characterized by an unusual thermal stability and resistance to degradation by steam. Nevertheless, with protracted use of such catalyst for reactions which inherently form steam, loss of catalytic activity does occur which is not recoverable by burning in air or by other recognized techniques for regeneration.

The present invention is based on the observation that certain cobalt-containing zeolite catalysts which have become deactive by prolonged exposure at high temperatures to steam and hydrocarbons, may be regenerated by contact with hydrogen sulfide, or other sulfur compounds.

Although the catalytic conversion of hydrocarbons in the presence of hydrogen sulfide or of sulfur compounds is described in U.S. Patents 3,173,858 and 3,175,967, the use of hydrogen sulfide for catalyst regeneration has not hitherto been described.

According to the present invention, there is provided a process for converting lower alcohols or their corresponding ethers into higher carbon-number hydrocarbons which comprises contacting such alcohols or ethers under conversion conditions with a catalyst comprising a crystalline zeolite and cobalt, characterized in that the zeolite has a silica/alumina mole ratio of at least 12 and a constraint index of 1 to 12, and that the catalyst has a cobaltous oxide/alumina mole ratio of 0.1 to 1.0, and is thereafter regenerated by contact with a sulfur compound which serves to convert at least a portion of the cobalt into cobalt sulfide at a temperature of 300 to 600°C.

The preferred sulfur compound for use in regenerating the catalyst is hydrogen sulfide.

The process of this invention utilizes a cobalt-impregnated or -exchanged crystalline zeolite composition as catalyst. In this process, a feed comprising lower alcohols or their corresponding ethers is contacted with the catalyst under conversion conditions, thereby converting the oxygenated compound to hydrocarbon and steam. After a protracted period of time, the catalyst, which becomes deactivated due to the high temperature exposure to steam, is contacted with hydrogen sulfide, restoring its catalytic activity, and the conversion of the oxygenated compound is resumed. This restoration of catalytic activity may be practiced one or more times, or until the hydrogen sulfide treatment is no longer effective in restoring catalytic activity.

The catalyst and process are particularly adapted to the conversion of the lower alcohols or their corresponding ethers to higher carbon-number hydrocarbons, i.e. to conversions which inherently produce steam.

The catalyst used in the process of this invention is prepared by compositing cobalt with a particular crystalline zeolite. Compositing may be effected by ion-exchange of the zeolite with a cobalt salt, by impregnation of the zeolite with a soluble cobalt compound, and by other means which lead to an intimate association of the cobalt with the zeolite. Regardless of the method of compositing, the catalyst composition of this invention is suitably substantially free of sodium, i.e. it has a sodium content less than about 0.5 wt. % computed as $Na_2O$. This is readily achieved, for example, by compositing the cobalt with the hydrogen form or the ammonium form of the zeolite, and by other

2

methods known to those skilled in the art. Regardless of the method of compositing, the catalyst also is required to have a $CoO/Al_2O_3$ (i.e. a cobalt oxide to zeolitic alumina molar ratio) of 0.1 to about 1.0.

The particular crystalline zeolites utilized herein have silica/alumina mole ratios of at least 12 and constraint indexes of 1 to 12. Although these zeolites have low alumina contents, i.e. high silica to alumina ratios, they are very active even when the silica to alumina ratio exceeds 30. The activity is surprising since catalytic activity is generally attributed to framework aluminum atoms and/or cations associated with these aluminum atoms. These zeolites retain their crystallinity for long periods in spite of the presence of steam at high temperature which induces irreversible collapse of the framework of other zeolites, e.g. of the X and A type. Furthermore, carbonaceous deposits, when formed, may be removed by burning at higher than usual temperatures to restore activity. These zeolites, used as catalysts, generally have low coke-forming activity and therefore are conducive to long times on stream between regenerations by burning with oxygen-containing gas such as air.

An important characteristic of the crystal structure of this class of zeolites is that it provides constrained access to and egress from the intracrystalline free space by virtue of having an effective pore size intermediate between the small pore Linde A and the large pore Linde X, i.e. the pore windows of the structure have about a size such as would be provided by 10-membered rings of oxygen atoms. It is to be understood, of course, that these rings are those formed by the regular disposition of the tetrahedra making up to the anionic framework of the crystalline aluminosilicate, the oxygen atoms themselves being bonded to the silicon or aluminum atoms at the centers of the tetrahedra. Briefly, the zeolites useful in this invention possess, in combination: a silica to alumina mole ratio of at least 12; and a structure providing constrained access to the crystalline free space.

The silica to alumina ratio may be determined by conventional analysis. This ratio is meant to represent, as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least about 30. Such zeolites, after activation, acquire an intracrystalline sorption capacity for normal hexane which is greater than that for water, i.e. they exhibit "hydrophobic" properties. It is believed that this hydrophobic character is advantageous in the present invention.

The zeolites useful in this invention have an effective pore size such as to freely sorb normal hexane. In addition, the structure must provide constrained access to larger molecules. It is sometimes possible to judge from a known crystal structure whether such constrained access exists. For example, if the only pore windows in a crystal are formed by 8-membered rings of oxygen atoms, then access by molecules of larger cross-section than normal hexane is excluded and the zeolite is not of the desired type. Windows of 10-membered rings are preferred, although in some instances excessive puckering of the rings or pore blockage may render these zeolites ineffective. 12-membered rings usually do not offer sufficient constraint to produce the advantageous conversions although the puckered 12-ring structure of TMA offretite shows constrained access. Other 12-ring structures may exist which, due to pore blockage or to other cause, may be operative.

Rather than attempt to judge from crystal structure whether or not a zeolite possesses the necessary constrained access to molecules larger than normal paraffins, a simple determination of the "Constraint Index" as herein defined may be made by passing continuously a mixture of an equal weight of normal hexane and 3-methylpentane over a small sample, approximately one gram or less, of zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 538°C for at least 15 minutes. The zeolite is then flushed with helium and the temperature is adjusted between 288°C and 510°C to give an overall conversion between 10% and 60%. The mixture of hydrocarbons is passed at 1 liquid hourly space velocity (i.e., 1 volume of liquid hydrocarbon per volume of zeolite per hour) over the zeolite with a helium dilution to give a helium to total hydrocarbon mole ratio of 4:1. After 20 minutes on stream, a sample of the effluent is taken and analyzed, most conveniently by gas chromatography, to determine the fraction remaining unchanged for each of the two hydrocarbons.

The "Constraint Index" is calculated as follows:

$$\text{Constraint Index} = \frac{\log 10 \text{ (fraction of n-hexane remaining)}}{\log 10 \text{ (fraction of 3-methylpentane remaining)}}$$

The Constraint Index approximates the ratio of the cracking rate constants for the two hydrocarbons. Zeolites suitable for the present invention are those having a Constraint Index of 1 to 12. Constraint Index (CI) values for some typical zeolites are:

3

**0018117**

| Zeolite | C.I. |
|---|---|
| ZSM-4 | 0.5 |
| ZSM-5 | 8.3 |
| ZSM-11 | 8.7 |
| ZSM-12 | 2 |
| ZSM-23 | 9.1 |
| ZSM-35 | 4.5 |
| ZSM-38 | 2 |
| TMA Offretite | 3.7 |
| Zeolite Beta | 0.6 |
| H-Zeolon (mordenite) | 0.4 |
| Rare earth-exchanged zeolite Y | 0.4 |
| Amorphous Silica-Alumina | 0.6 |
| Erionite | 38 |

The above-described Constraint Index is an important and even critical definition of those zeolites which are useful in the instant invention. The very nature of this parameter and the recited technique by which it is determined, however, admit of the possibility that a given zeolite can be tested under somewhat different conditions and thereby have different Constraint Indexes. Constraint Index seems to vary somewhat with severity of operation (conversion) and the presence or absence of binders. Therefore, it will be appreciated that it may be possible to so select test conditions to establish more than one value in the range of 1 to 12 for the Constraint Index of a particular zeolite. Such a zeolite exhibits the constrained access as herein defined and is to be regarded as having a Constraint Index of 1 to 12. Also contemplated herein as having a Constraint Index of 1 to 12 and therefore within the scope of the zeolites used herein are those zeolites which, when tested under two or more sets of conditions within the above-specified ranges of temperature and conversion, produce a value of the Constraint Index slightly less than 1, e.g. 0.9, or somewhat greater than 12, e.g. 14 or 15, with at least one other value of 1 to 12. Thus, it should be understood that the Constraint Index value as used herein is an inclusive rather than an exclusive value. That is, a zeolite when tested by any combination of conditions within the testing definition set forth hereinabove to have a Constraint Index of 1 to 12 is intended to be included in the instant catalyst definition regardless that the same identical zeolite tested under other defined conditions may give a Constraint Index value outside of 1 to 12.

The class of zeolites defined herein is exemplified by ZSM-5, ZSM-11, ZSM-12, ZSM-23, ZSM-35, ZSM-38, and other similar materials. U.S. Patent 3,702,886 describing and claiming ZSM-5 is incorporated herein by reference.

ZSM-11 is more particularly described in U.S. Patent 3,709,979, the entire content of which is incorporated herein by reference.

ZSM-12 is more particularly described in U.S. Patent 3,832,449, the entire content of which is incorporated herein by reference.

ZSM-23 is more particularly described in U.S. Patent 4,076,842, the entire content of which is incorporated herein by reference.

ZSM-35 is more particularly described in U.S. Patent 4,016,245, the entire content of which is incorporated herein by reference.

ZSM-38 is more particularly described in U.S. Patent 4,046,859, the entire content of which is incorporated herein by reference.

The specific zeolites described, when prepared in the presence of organic cations, are substantially catalytically inactive, possibly because the intracrystalline free space is occupied by organic cations from the forming solution. They may be activated by heating in an inert atmosphere at 538°C for one hour, for example, followed by base exchange with ammonium salts followed by calcination at 538°C in air. The presence of organic cations in the forming solution may not be absolutely essential to the formation of this type zeolite; however, the presence of these cations does appear to favor the formation of this class of zeolite. More generally, it is desirable to activate this type catalyst by base exchange with ammonium salts followed by calcination in air at about 538°C for from about 15 minutes to about 24 hours.

Natural zeolites may sometimes be converted to this type zeolite catalyst by various activation procedures and other treatments such as base exchange, steaming, alumina extraction and calcination, in combinations. Natural minerals which may be so treated include ferrierite, brewsterite, stilbite, dachiardite, epistilbite, heulandite, and clinoptilolite.

In a preferred aspect of this invention, the zeolites are selected as those having a crystal framework density, in the dry hydrogen form, of not less than 1.6 grams per cubic centimeter. It has been found that zeolites which satisfy all three of these criteria are most desired for several reasons. When hydrocarbon products or by-products are catalytically formed, for example, such zeolites tend to maximize the production of gasoline boiling range hydrocarbon products. Therefore, the preferred zeolites of this invention are those having a Constraint Index as defined above of 1 to 12, a silica to

4

alumina ratio of at least 12 and a dried crystal density of not less than 1.6 grams per cubic centimeter. The dry density for known structures may be calculated from the number of silicon plus aluminum atoms per 1000 cubic Angstroms, as given, e.g., on Page 19 of the article on Zeolite Structure by W. M. Meier. This paper, the entire contents of which are incorporated herein by reference, is included in "Proceedings of the Conference on Molecular Sieves, London, April 1967", published by the Society of Chemical Industry, London, 1968. When the crystal structure is unknown, the crystal framework density may be determined by classical pyknometer techniques. For example, it may be determined by immersing the dry hydrogen form of the zeolite in an organic solvent which is not sorbed by the crystal. Or, the crystal density may be determined by mercury porosimetry, since mercury will fill the interstices between crystals but will not penetrate the intracrystalline free space. It is possible that the unusual sustained activity and stability of this class of zeolites is associated with its high crystal anionic framework density of not less than about 1.6 grams per cubic centimeter. This high density must necessarily be associated with a relatively small amount of free space within the crystal, which might be expected to result in more stable structures. This free space, however, is important as the locus of catalytic activity.

Crystal framework densities of some typical zeolites including some which are not within the purview of this invention are:

| Zeolite | Void volume | Framework density |
|---|---|---|
| Ferrierite | 0.28 cm$^3$/cm$^3$ | 1.76 g/cm$^3$ |
| Mordenite | .28 | 1.7 |
| ZSM-5, -11 | .29 | 1.79 |
| Dachiardite | .32 | 1.72 |
| L | .32 | 1.61 |
| Clinoptilolite | .34 | 1.71 |
| Laumontite | .34 | 1.77 |
| ZSM-4 (Omega) | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

When synthesized in the alkali metal form, the zeolite is conveniently converted to the hydrogen form, generally by intermediate formation of the ammonium form as a result of ammonium ion exchange and calcination of the ammonium form to yield the hydrogen form. In addition to the hydrogen form, other forms of the zeolite wherein the original alkali metal has been reduced to less than about 0.5 percent by weight may be used. Thus, the original alkali metal of the zeolite may be replaced by ion exchange with cobalt, but other suitable ions of Groups IB to VIII of the Periodic Table, including, by way of example, nickel, copper, zinc, palladium, calcium or rare earth metals, also may be present.

In practicing the desired conversion process, it may be desirable to incorporate the above-described crystalline aluminosilicate zeolite in another material resistant to the temperature and other conditions employed in the process. Such matrix materials include synthetic or naturally occurring substances as well as inorganic materials such as clay, silica and/or metal oxides. The relative proportions of zeolite component and inorganic matrix on an anhydrous basis may vary widely with the zeolite content ranging from between about 1 to about 99 percent by weight and more usually in the range of about 5 to about 80 percent by weight of the dry composite.

It has been found that certain matrix materials, particularly alumina, detract from the stability of the catalyst of this invention. Therefore, where a matrix or binder is used with the zeolite, it is preferred that this matrix or binder has an alumina content of less than about 10 wt.%. Silica is a preferred binder.

The particular zeolite hereinabove described is composited with sufficient cobalt to form a composition having, on an anhydrous basis, a CoO/Al$_2$O$_3$ molar ratio of 0.1 to 1.0. This may be achieved by base exchange of the ammonium or hydrogen zeolite with cobalt acetate, cobalt nitrate, or other soluble cobalt salts. Compositing by impregnation also is effective. Preferred zeolites are ZSM-5, ZSM-11, ZSM-23, ZSM-35 and ZSM-38, with ZSM-5 particularly preferred.

The catalyst used in this invention preferably has a minimum catalytic activity regardless of which zeolite is selected and of its cobalt content. This activity is precisely specified by determination of the "alpha value" of the catalyst, which measures its activity for the cracking of n-hexane. The alpha value

5

is to be determined in accordance with the method set forth by P. B. Weisz and J. N. Miale in "Journal of Catalysis", Vol. 4, No. 4, August 1969, pp. 527—529, which description is herein incorporated by reference. The alpha value, for purposes of this invention, is to be at least 10, and preferably in the range of 10 to 60.

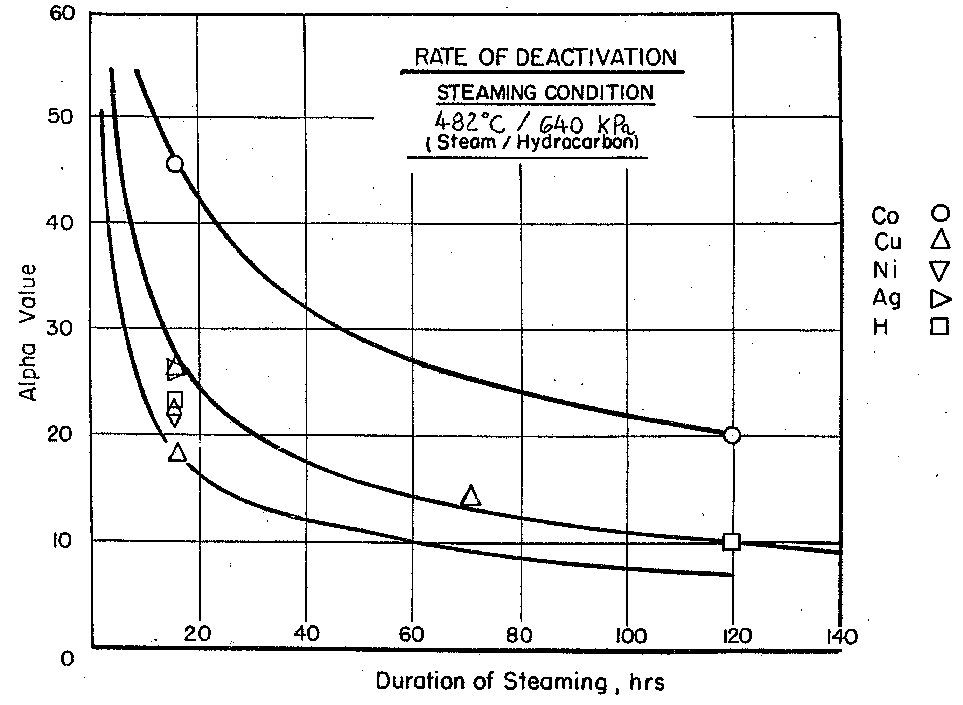

The resistance to loss of catalytic activity of the cobalt catalyst used in this invention compared with other metal forms when exposed to a mixture of steam and hydrocarbons is illustrated by the drawing.

While it is not known precisely why the catalyst composition of this invention behaves in the manner it does, nonetheless, certain observations and speculations have been made which may be useful for the understanding of its activity. With perhaps the exception of the alkaline metals, such as sodium, the metal-exchanged forms of certain zeolites have been found more resistant to degradation in pure steam than the hydrogen forms of the zeolites. Thus, utilizing ZSM-5 as an example, the cobalt, copper, nickel and the zinc forms of this zeolite lose their catalytic activity when exposed to pure steam at high temperature at a slower rate than the hydrogen form. Thus, it may be speculated that cobalt and copper and other metal cations protect the alumina sites from hydrolysis.

In a steam atmosphere that contains hydrocarbons, however, copper does not appear to be effective, while cobalt is. The persistent pink color of the cobalt catalyst suggests that the cobalt ion is not reduced from its divalent metal state in the adverse environment and that its persistence in that form in a catalytic environment is needed to protect the alumina sites from degradation. On the other hand, it has been noted that the copper form of the zeolite rapidly turns black in steam when hydrocarbons are present, suggesting that it is reduced from the divalent state and loses its protective function. In brief, the cationic forms of the zeolites appear to fall into two categories. In the first category are those non-reducible cations such as cobalt which retain their ionic form in a high temperature hydrocarbon-steam atmosphere, and in the second category are all the other cations, which are reduced in such an atmosphere and therefore exhibit little if any protective function.

Preferred feeds for the process of the invention are lower aliphatic alcohols containing up to four carbon atoms, their simple or mixed ethers, or mixtures thereof. Such alcohols or ether feeds are converted to higher carbon-number hydrocarbons with inherent formation of steam. Thus produced steam will normally result in a partial steam pressure of at least 10 kPa, and preferably from 10 to about 1010 kPa. Depending on feed and reaction conditions, the process is made to produce predominantly olefins, or olefins and aromatic hydrocarbons in the gasoline boiling range, or a liquid hydrocarbon mixture useful as high octane gasoline.

For the purpose of this invention it is advantageous to use a feed substantially free of sulfur, i.e., a feed that contains less than about 75 parts per million of sulfur, and preferably less than 10 parts per million.

In general, the catalytic conversion of the process of this invention is conducted at a liquid hourly space velocity (LHSV) of from 0.5 to about 100, a temperature from about 275°C to 600°C, and at a total pressure of from about 50 to 5066 kPa.

The process of this invention may be operated with a fixed stationary bed of catalyst, a fixed fluid bed, a fixed ebullated bed, or a transport bed. Accumulations of coke that may occur with detriment to the catalyst activity or selectivity may be burned off the catalyst at elevated temperatures in the usual fashion. Deactivation due to long term exposure to steam is reversed by contacting the catalyst with hydrogen sulfide at a temperature of 300° to 600°C and in an amount effective to substantially increase the alpha value of the deactivated catalyst thereby regenerating the catalyst. The effect of this contact with hydrogen sulfide will generally be to convert at least some of the cobalt to cobalt sulfide. Hydrogen sulfide treatment may be carried out prior to burning to remove coke, but is most preferably carried out subsequent to such burning if coke-removal is required. Obvious variants of the hydrogen sulfide treatment, such as contact with thiophene, elementary sulfur, mercaptans, or, in effect any sulfur compound which will serve to convert a portion of the cobalt to cobalt sulfide, is contemplated as within the scope of this invention.

The following Example illustrates the invention.

Example

15 grams of an ammonium form ZSM-5 ($NH_4$—ZSM-5) having a $SiO_2/Al_2O_3$ molar ratio of about 70 was base exchanged with a solution of 29 grams of cobalt dinitrate hexahydrate in 250 ml of water to provide a cobalt-ammonium ZSM-5 containing cobalt in an amount equal to 0.89 wt.% cobalt oxide (CoO). This preparation was impregnated with an additional amount of cobalt as the dinitrate in water and dried and calcined. The final catalyst contained about 2.5 wt.% CoO, had a $CoO/Al_2O_3$ molar ratio of about 1 and considerably less than 0.5 wt.% $Na_2O$.

This catalyst was subjected to accelerated aging by contact for 120 hours at 482°C and about 650 kPa pressure provided by about an equimolar mixture of n-hexane and steam. At the end of this cycle it had an alpha-value of about 20 determined by n-hexane cracking.

A portion of the aged catalyst was used to catalyze the conversion of methanol into hydrocarbons. After service in this conversion, it was regenerated by burning in air and contact with $H_2S$ at 510°C. Regeneration doubled the catalytic activity for methanol conversion.

**0018117**

## Claims

1. A process for converting lower alcohols or their corresponding ethers into higher carbon-number hydrocarbons which comprises contacting such alcohols or ethers under conversion conditions with a catalyst comprising a crystalline zeolite and cobalt, characterized in that the zeolite has a silica/alumina mole ratio of at least 12 and a constraint index of 1 to 12, and that the catalyst has a cobaltous oxide/alumina mole ratio of 0.1 to 1.0, and is thereafter regenerated by contact with a sulfur compound which serves to convert at least a portion of the cobalt into cobalt sulfide at a temperature of 300 to 600°C.

2. A process according to claim 1, wherein the zeolite is ZSM-5, ZSM-11, ZSM-12, ZSM-23, ZSM-35 or ZSM-38.

3. A process according to claim 1 or claim 2, wherein the zeolite is substantially free of alkali metal and has an alpha value of at least 10.

4. A process according to any one of claims 1 to 3, wherein the zeolite is composited in a substantially alumina-free binder or matrix.

5. A process according to any one of claims 1 to 4, wherein the sulfur compound is hydrogen sulfide.

6. A process according to any one of claims 1 to 5, wherein the feed is an alcohol having up to four carbon atoms or a simple or mixed ether thereof.

7. A process according to any one of claims 1 to 6, wherein the feed is substantially free of sulfur.

## Revendications

1. Procédé pour la conversion d'alcools inférieurs ou des éthers correspondants en hydrocarbures ayant un nombre d'atomes carbone plus élevé qui comprend le contact de tels alcools ou éthers dans des conditions de conversion avec un catalyseur comprenant une zéolite cristalline et du cobalt, caractérisé en ce que la zéolite a un rapport molaire silice/alumine d'au moins 12 et un indice de contrainte de 1 à 12, et en ce que le catalyseur a un rapport molaire oxyde cobalteux/alumine de 0,1 à 1,0, et est ensuite régénéré par contact avec un composé soufré qui sert à transformer au moins une portion du cobalt en sulfure de cobalt à une température de 300 à 600°C.

2. Procédé selon la revendication 1, dans lequel la zéolite est la ZSM-5, ZSM-11, ZSM-12, ZSM-23, ZSM-35 ou ZSM-38.

3. Procédé selon la revendication 1 ou la revendication 2, dans lequel la zéolite est pratiquement dépourvue de métal alcalin et a une valeur $\alpha$ d'au moins 10.

4. Procédé selon l'une quelconque des revendications 1 à 3, dans lequel la zéolite est combinée dans un liant ou une matrice, essentiellement dépourvu d'alumine.

5. Procédé selon l'une quelconque des revendications 1 à 4, dans lequel le composé soufré est l'acide sulfhydrique.

6. Procédé selon l'une quelconque des revendications 1 à 5, dans lequel l'alimentation est un alcool ayant jusqu'à 4 atomes de carbone ou un éther simple ou mixte correspondant.

7. Procédé selon l'une quelconque des revendications 1 à 6, dans lequel l'alimentation est pratiquement dépourvue de soufre.

## Patentansprüche

1. Verfahren zur Umwandlung von niedrigeren Alkoholen oder von deren entsprechenden Äthern in Kohlenwasserstoffe mit höherer Kohlenstoffanzahl, wobei solche Alkohole oder Äther unter Umwandlungsbedingungen mit einem Katalysator in Berührung gebracht werden, der einen kirstallinen Zeolithen un Kobalt enthält, dadurch gekennzeichnet, daß der Zeolith ein Siliciumdioxid/Aluminiumoxid-Molverhältnis von wenigstens 12 und einen Zwangsindex von 1 bis 12 aufweist und daß der Katalysator ein Kobaltoxid/Aluminiumoxid-Molverhältnis von 0,1 bis 1,0 hat, und anschließend eine Regenerierung durch Inberührungbringen mit einer Schwefelverbindung zwecks Umwandlung wenigstens eines Teils des Kobalts in Kobaltsulfid bei einer Temperatur von 300 bis 600°C erfolgt.

2. Verfahren nach Anspruch 1, dadurch gekennzeichnet, daß der Zeolith ZSM-5, ZSM-11, ZSM-12, ZSM-23, ZSM-35 oder ZSM-38 ist.

3. Verfahren nach Anspruch 1 oder 2, dadurch gekennzeichnet, daß der Zeolith im wesentlichen frei von Alkalimetall ist und einen alpha-Wert von wenigstens 10 hat.

4. Verfahren nach einem der Ansprüche 1 bis 3, dadurch gekennzeichnet, daß der Zeolith mit einem im wesentlichen aluminiumoxidfreien Bindemittel oder Matrixmaterial zusammengesetzt ist.

5. Verfahren nach einem der Ansprüche 1 bis 4, dadurch gekennzeichnet, daß die Schwefelverbindung Schwefelwasserstoff ist.

6. Verfahren nach einem der Ansprüche 1 bis 5, dadurch gekennzeichnet, daß das Einsatzmaterial ein Alkohol mit bis zu vier Kohlenstoffatomen oder ein einfacher oder gemischter Äther davon ist.

7. Verfahren nach einem der Ansprüche 1 bis 6, dadurch gekennzeichnet, daß das Zufuhrmaterial im wesentlichen frei von Schwefel ist.

RATE OF DEACTIVATION

STEAMING CONDITION

482°C / 640 kPa
( Steam / Hydrocarbon)

Co    ○
Cu    △
Ni    ▽
Ag    ▷
H     □

Alpha Value

Duration of Steaming , hrs